# EUROPÄISCHE PATENTSCHRIFT

(11) **EP 2 877 219 B1**
(45) Veröffentlichungstag und Bekanntmachung des Hinweises auf die Patenterteilung: **11.05.2016**
(21) Anmeldenummer: 13737621.6
(22) Anmeldetag: 18.07.2013
(51) Int. Cl.: A61M 1/02, A61M 1/34, A61M 39/12

(54) **MOBILES SYSTEM ZUR MITTELS SCHWERKRAFT ERFOLGENDEN AUFTEILUNG VON SPENDERBLUT**
MOBILE SYSTEM TO SEPARATE DONATED BLOOD USING GRAVITY
SYSTÈME MOBILE DESTINÉ À SÉPARER LE SANG DU DONNEUR PAR GRAVITÉ

(30) Priorität: 25.07.2012 EP 12177871
(43) Veröffentlichungstag der Anmeldung: 03.06.2015
(73) Patentinhaber: 3M Innovative Properties Company, St. Paul, MN 55133-3427 (US)
(72) Erfinder: MÜLLER, Roland, 6301 Zug (CH); VON HARTEN, Bodo, 42349 Wuppertal (DE)
(74) Vertreter: CPW GmbH
(86) Internationale Anmeldenummer: PCT/EP2013/065208
(87) Internationale Veröffentlichungsnummer: WO 2014/016198

(56) Entgegenhaltungen:
- EP-A1- 2 277 573
- WO-A1-2008/070220
- WO-A2-2007/012321
- WO-A2-2008/083965
- DE-A1-102010 030 238
- DE-U1- 29 516 471
- US-A1- 2010 137 826

## Beschreibung

Die Erfindung betrifft ein mobiles System zur mittels Schwerkraft erfolgenden Aufteilung von Spenderblut in Erythrozytenkonzentrat und Plasma mit einem als Vorratsbehälter für das aufzutrennende Blut dienenden ersten Beutel und zumindest einem zweiten und einem dritten Beutel zur Aufnahme von Erythrozytenkonzentrat bzw. Plasma, sowie mehreren Schläuchen zur Verbindung der aus Kunststoffmaterial bestehenden flexiblen Beutel mit einem eine Trennmembran in Form eines Hohlfaserbündels aufweisenden Separatormodul.

Bei der im Regelfall verwendeten Methode zur Aufteilung von Spenderblut in Erythrozytenkonzentrat und Plasma erfolgt die Blutseparation durch Zentrifugation. Dabei wird in üblicher Weise ein Vierfachbeutelsystem verwendet, wobei die Beutel steril über Schläuche verbunden sind. Der im Rahmen einer Spende mit Vollblut gefüllte erste Beutel steht über einen Leukozytenfilter mit dem zweiten Beutel zur Aufnahme von gefiltertem Vollblut in Verbindung. Ist dieser zweite Beutel mit gefiltertem Blut gefüllt, wird er entlüftet und verschweißt.

Die Zentrifugation erfolgt in einem Rotator, wobei Rotationsgeschwindigkeit, Zentrifugationsdauer und Temperatur durch die zu separierenden Blutkomponenten und das verwendete Beutelsystem bestimmt werden. Die zentrifugierte Vollblutkonserve wird in einen Separator eingebracht, welcher beispielsweise über optische Detektoren die Trennschicht zwischen Plasma und Zellen ermittelt und diese dann jeweils in die verbundenen Beutel abpresst.

Dieses Standardverfahren der Blutkomponentenherstellung stellt zwar ein bewährtes Verfahren dar, ist aber auch mit mehreren Nachteilen verbunden.

Da für die Durchführung der Zentrifugation und Separation unabdingbar elektrische Energie benötigt wird, kann dieses Standardverfahren der Blutkomponentenherstellung nur bei Vorhandensein einer entsprechenden Energiequelle durchgeführt werden.

Neben den hohen Anschaffungs- und Unterhaltskosten, die mit diesem bekannten System verbunden sind, ist nachteilig, dass die praktische Durchführung der Blutseparation nur durch geschultes Personal erfolgen kann. Hinzu kommt, dass aufgrund der hohen Rotationsgeschwindigkeiten rote Blutzellen beim Aufprall an die Wandung der Zentrifuge zerplatzen und ihren Inhalt an das umgebende Plasma absondern können. Da diese roten Blutkörperchen neben dem Hämoglobin und Zellfragmenten auch giftige Substanzen und Krankheitserreger enthalten können, müssen diese gefährlichen Inhalte vor der Weiterverarbeitung des Plasmas extrahiert werden, was zusätzliche Arbeitsschritte und Kosten verursacht.

Ungünstig ist ferner, dass die in entsprechend ausgestatteten, zentralen Einrichtungen durchgeführte Herstellung von Blutkonserven häufig mit einer Zeitverzögerung verbunden ist, wenn Blutspenden in mobilen Spendestationen ausgeführt und die Spenden über größere Strecken zu dem entsprechendem Zentrum transportiert werden müssen, bevor dort die Fraktionierung in Blutkomponenten durchgeführt werden kann.

Unter der Bezeichnung "ErySep Classic" der Firma Lmb Technologie GmbH ist bereits ein mobiles System zur mittels Schwerkraft erfolgenden Aufteilung von Spenderblut in Erythrozytenkonzentrat und Plasma der eingangs angeführten Art bekannt. Dieses bekannte, von einer äußeren Energiequelle unabhängige System hat grundsätzlich den Vorteil, dass es die Separation von Spenderblut ohne die Notwendigkeit herkömmlicher Logistik ermöglicht, denn es besteht kein Bedarf an Elektrizität und elektronischen Geräten wie Zentrifugen, Plasmaextraktoren und dergleichen.

Bei diesem bekannten System sind die verschiedenen benötigten Beutel mit dem Separatormodul über Schläuche verbunden, die mit den Anschlüssen des Separatormoduls fest verbunden, insbesondere verklebt oder verschweißt sind, so dass dieses Gesamtsystem als Einheit sterilisiert werden muss. Ergibt sich bei dem stets durchzuführenden Test des Spenderbluts aus dem Vorspenderbeutel eine Unbrauchbarkeit des Spenderbluts, dann hat dies zur Folge, dass das Gesamtsystem, bestehend aus Beuteln, Verbindungschläuchen und Separatormodul, unbrauchbar wird und demgemäß die zur Einmalverwendung bestimmte Gesamteinheit entsorgt werden muss. Ähnliche Systeme, die jeweils einen Blutbeutel zur Aufnahme des Spenderbluts, einen Separatormodul, Beutel für Erythrozytenkonzentrat und Plasma sowie die diese Elemente verbindenden Schläuche umfassen, sind auch bereits aus der DE 295 16 471 U1, der EP 2 277 573 A1, der DE 10 2010 030238 A1 und der WO 2007/012321 A2 bekannt.

Aufgabe der vorliegenden Erfindung ist es, eine wesentliche Verbesserung des bisher bekannten Systems zur mittels Schwerkraft erfolgenden Aufteilung von Spenderblut in Erythrozytenkonzentrat und Plasma zu erreichen, und zwar sowohl in wirtschaftlicher Hinsicht als auch im Hinblick auf die Qualität der erhaltenen Blutkomponenten und des angestrebten hohen Hämatokritwertes.

Diese Aufgabe wird nach der Erfindung im Wesentlichen dadurch gelöst, dass das System in mindestens ein erstes und ein zweites steril verpacktes, individuelles Teilsystem aufgeteilt ist,
dass das mindestens eine erste Teilsystem die einzelnen Beutel und zugehörige Verbindungsschläuche und das zweite Teilsystem das Separatormodul umfasst, und
dass zur Verbindung der Schläuche mit dem Separatormodul Male-/ Female-Konnektoren, insbesondere Luer-Lock-Konnektören, vorgesehen sind, deren einer Teil jeweils im Gehäuse des Separatormoduls fixiert bzw. integriert und deren komplementärer Teil mit dem Ende des jeweiligen Schlauchs verbunden ist.

Damit wird beispielsweise ein Two-in-One-Filterungs- und Separationssystem für den einmaligen Gebrauch zur Verfügung gestellt, das in besonders wirtschaftlicher Weise eine sichere Separation der Blutkomponenten ermöglicht, wobei kein Bedarf an Elektrizität und elektronischen Geräten und auch kein Bedarf an speziell ausgebildetem Personal besteht und die separierten Komponenten zum sofortigen Gebrauch zur Verfügung stehen.

Durch die Aufteilung des Gesamtsystems in mindestens zwei definierte Teilsysteme werden nicht nur in der Praxis wichtige Handling-Vorteile erzielt, sondern auch vermieden, dass bei nicht verwertbarem Spenderblut nicht das Gesamtsystem, sondern nur das preiswerte mindestens eine erste Teilsystem entsorgt werden muss. Dieser wirtschaftlich wesentliche Effekt hat gerade dann besondere Bedeutung, wenn mit einem hohen Anteil an verseuchtem Blut, z.B. in Malariagebieten, gerechnet werden muss.

Ein weiterer Vorteil der Aufteilung besteht darin, dass das zweite Teilsystem, d.h. das Separatormodul, mit verschiedenen Ausführungen des mindestens einen ersten Teilsystem kombiniert werden kann. So ist beispielsweise nicht für alle Anwendungen und für alle Regionen der Welt ein Leukozytenfilter zwischen Primärbeutel für Spenderblut und Separatormodul erforderlich. Durch die Aufteilung des Gesamtsystems in mindestens zwei definierte Teilsysteme kann dann das zweite Teilsystem mit dem Separatormodul wahlweise mit ersten Teilsystemen kombiniert werden, die einen Leukozytenfilter umfassen, oder mit ersten Teilsystemen ohne Leukozytenfilter. Auf diese Weise kann die Wirtschaftlichkeit des Gesamtsystems erhöht werden.

Das Grundkonzept der erfindungsgemäßen Aufteilung des Gesamtsystems ermöglicht es in vorteilhafter Weise, die verpackten Teilsysteme mittels unterschiedlicher Verfahren zu sterilisieren, wobei für das mindestens eine erste Teilsystem die in der Praxis bewährte Dampfsterilisation verwendet werden kann und das zweite Teilsystem mit dem Separatormodul beispielsweise durch Gamma- oder Betastrahlung sterilisiert wird. Diese Vorgehensweise führt zu einem Höchstmaß an Sicherheit hinsichtlich der Anforderungen an die Sterilität des Gesamtsystems. Dazu trägt auch bei, dass in den steril verpackten Teilsystemen die offenen Teil-Konnektoren durch abnehmbare Passkappen verschlossen sind, so dass das Zusammenfügen der Teilsysteme zu einem Gesamtsystem in einer in der Medizintechnik seit langer Zeit bewährten Weise erfolgen kann und dabei sterile oder zumindest weitgehend sterile Verbindungsstellen gewährleistet werden können.

Das vorliegende mobile System zur mittels Schwerkraft erfolgenden Aufteilung von Spenderblut in Erythrozytenkonzentrat und Plasma ist in mindestens ein erstes und ein zweites steril verpacktes, individuelles Teilsystem aufgeteilt. Das zweite Teilsystem umfasst das Separatormodul. Die einzelnen Beutel und zugehörigen Verbindungsschläuche sind vom mindestens einen ersten Teilsystem umfasst. Dabei können die einzelnen Beutel und Verbindungsschläuche sowie weitere erforderliche oder optionale Bestandteile des mobilen Systems mit Ausnahme des Separatormoduls von einem einzelnen ersten Teilsystem umfasst sein. Es können jedoch auch mehrere erste Teilsysteme vorliegen, auf die die einzelnen Beutel und Verbindungsschläuche sowie weitere erforderliche oder optionale Bestandteile des mobilen Systems aufgeteilt sein. So kann in einer Ausführungsform das mobile System zur mittels Schwerkraft erfolgenden Aufteilung von Spenderblut zwei erste Teilsysteme aufweisen, wobei das eine dieser Teilsysteme beispielsweise den als Vorratsbehälter für das aufzutrennende Blut dienenden ersten Beutel, d.h. den Primärbeutel für Spenderblut, sowie einen eventuell vorhandenen Vorspenderbeutel für Testzwecke, eine Spenderkanüle sowie zugehörige Verbindungsschläuche umfasst. Das weitere erste Teilsystem kann dann den zweiten und den dritten Beutel zur Aufnahme von Erythrozytenkonzentrat bzw. Plasma sowie die hierzu zugehörigen Verbindungsschläuche umfassen. Auch eine Aufteilung in drei oder mehr erste Teilsysteme ist möglich, die dann eine weitere Aufteilung der Bestandteile des Gesamtsystems in Untergruppen ermöglicht.

Bevorzugt ist jedoch eine Ausführungsform, bei der das mobile System zur mittels Schwerkraft erfolgenden Aufteilung von Spenderblut in ein einzelnes erstes und ein zweites steril verpacktes, individuelles Teilsystem aufgeteilt ist und dass das einzelne erste Teilsystem die Gesamtheit der einzelnen Beutel und zugehörigen Verbindungsschläuche umfasst. Eine solche Ausführungsform bietet Vorteile hinsichtlich der Handhabung der einzelnen Teilsysteme und deren Zusammenbau.

Insbesondere bei der zuvor genannten Ausführungsform sind im steril verpackten ersten Teilsystem die Konnektoranschlüsse der Zu- und Ablaufschläuche der Beutel mit einem Y-Konnektor über Male-/Female-Verbindungen zusammengeführt, so dass die Dampfsterilisation dieses Teilsystems bei miteinander verbundenen Beuteln erfolgen kann. Werden die beiden Teilsysteme zu einem Gesamtsystem zusammengefügt, werden die Verbindungen mit dem Y-Konnektor gelöst und die erforderlichen Male-/ Female-Konnektorverbindungen hergestellt.

Nach einer besonders vorteilhaften Ausgestaltung der Erfindung ist in dem rohrförmigen, sich in Richtung des Bodens vorzugsweise leicht verjüngendem Gehäuse des Separatormoduls ein U-förmig angeordnetes Bündel von Membran-Hohlfasern vorgesehen, wobei das Gehäuse des Separatormoduls bodenseitig einen Auslassstutzen für das Plasma und deckelseitig einen mit den Einlässen des Hohlfaserbündels verbundenen Zulaufstutzen für das zu trennende Spenderblut sowie einen Auslassstutzen für das Erythrozytenkonzentrat aufweist. Dabei ist sowohl in fertigungstechnischer Hinsicht als auch im Hinblick auf die angestrebte hohe Funktionssicherheit von besonderer Bedeutung, dass unmittelbar in dem j eweiligen Stutzen die für die sterilen oder zumindest weitgehend sterilen Schlauchanschlüsse erforderlichen Teil-Konnektoren insbesondere durch Verkleben oder Verschweißen fixiert sind oder bevorzugt diese Teil-Konnektoren durch Gestaltung einer speziellen Gussform direkt an die Stutzen angegossen werden, also integraler Bestandteil der Stutzen sind, wobei in letzterem Falle eine separate Handhabung der Konnektoren und der Arbeitsschritt des Verklebens oder Verschweißens vermieden werden kann.

Für die zwangsläufig richtige Verbindung der jeweiligen Anschlüsse und Schläuche über die Konnektoren ist es bevorzugt, wenn der bodenseitige Auslassstutzen des Separatormoduls einen von den deckelseitigen Stutzen verschiedenen Teil-Konnektor aufweist. In einer Ausführungsform ist dabei von Vorteil, dass der bodenseitige Auslassstutzen des Separatormoduls einen Male-Konnektor aufweist und den deckelseitigen Stutzen Female-Konnektoren zugeordnet sind, die einerseits mit einem Male-Konnektor des zum Spenderbeutel führenden Schlauches und andererseits mit einem Male-Konnektor des zum Beutel für das Erythrozytenkonzentrat führenden Schlauchs kuppelbar sind. Entsprechend weist der zum Beutel für Plasma führende Schlauch einen Female-Konnektor auf und ist damit mit dem Male-Konnektor des bodenseitigen Auslassstutzens des Separatormoduls kuppelbar. Gemäß einer weiteren Ausführungsform ist es von Vorteil, dass der bodenseitige Auslassstutzen des Separatormoduls einen Female-Konnektor aufweist, der mit einem Male-Konnektor des zum Plasmabeutel führenden Schlauches kuppelbar ist. Den deckelseitigen Stutzen des Separatormoduls sind in dieser Ausführungsform Male-Konnektoren zugeordnet, die einerseits mit einem Female-Konnektor des zum Spenderbeutel führenden Schlauches und andererseits mit einem Female-Konnektor des zum Beutel für das Erythrozytenkonzentrat führenden Schlauchs kuppelbar sind.

In einer weiteren vorteilhaften Ausführungsform ist an den deckelseitigen Stutzen zur Ankupplung des Beutels für das Erythrozytenkonzentrat ein Anschlussstück zur Druckanpassung ankuppelbar oder angekuppelt, an das wiederum der zum Beutel für das Erythrozytenkonzentrat führenden Schlauch ankuppelbar ist.

Das gesonderte Anschlussstück zur Druckanpassung mit beidendig vorgesehenen Teil-Konnektoren kann Bestandteil des steril verpackten zweiten Teilsystems sein. Von Vorteil ist eine Ausführungsform, bei der das gesonderte Anschlussstück zur Druckanpassung mit beidendig vorgesehenen Teil-Konnektoren Bestandteil des steril verpackten mindestens einen ersten Teilsystems ist. Dies erlaubt die Bereitstellung von mobilen Systemen zur Aufteilung von Spenderblut mittels Schwerkraft, bei denen das zweite Teilsystem stets gleich ausgeführt ist und bedarfsangepasst mit ersten Teilsystemen gekoppelt wird, die je nach Anforderung ein Anschlussstück zur Druckanpassung und/oder auch z.B. einen dem Primärbeutel für Spenderblut nachgeschalteten Leukozytenfilter umfassen können.

Das Anschlussstück zur Druckanpassung kann ein Leitungsabschnitt sein, der einen im Vergleich zu den übrigen Verbindungsschläuchen verringerten Innendurchmesser aufweist, wobei dieser Innendurchmesser in Verbindung mit der Länge dieses Leitungsabschnitts so gewählt ist, dass infolge des dadurch in der Anwendung erhöhten Druckabfalls ein besonders hoher Hämatokritwert im Erythrozytenkonzentrat erhalten wird. Das Anschlussstück kann ein entsprechend ausgestaltetes Leitungsstück in Gestalt eines gesonderten Schlauchabschnitts oder in Form eines Rohres oder einer Kapillare sein. Des weiteren kann das Anschlussstück ein mit einem einstellbaren Ventil versehenes Leitungsstück sein. Es sind auch Ausführungsformen möglich, wie sie als Druckeinstellvorrichtung in der WO 2011/157822 beschrieben werden, auf deren diesbezügliche Offenbarung ausdrücklich Bezug genommen wird.

In einer vorteilhaften Ausführungsform ist das Schlauchstück zwischen Primärbeutel für Spenderblut und Teil-Konnektor zur Kupplung mit dem deckelseitigen Einlassstutzen des Separatormoduls bzw., bei Vorhandensein eines Leukozytenfilters zwischen Primärbeutel und Separatormodul, das Schlauchstück zwischen Leukozytenfilter und Teil-Konnektor zur Kupplung mit dem deckelseitigen Einlassstutzen des Separatormoduls in Bezug auf seinen Innendurchmesser und seine Länge so ausgelegt, dass in der Anwendung der Druckverlust durch dieses Schlauchstück hoch genug ist, um ein zu schnelles Einfließen des Bluts in den Separatormodul und damit eine Hämolyse des Blutes zu verhindern. Hierdurch wird eine Vereinfachung der Handhabung des Gesamtsystems in der Anwendung erreicht, beispielsweise auch dadurch, dass auf eine sonst übliche Rollklemme zur Einstellung des Volumenstroms verzichtet werden kann.

Weitere vorteilhafte Merkmale und Besonderheiten der Erfindung sind in den Unteransprüchen beschrieben und werden auch anhand des nachfolgend erläuterten Ausführungsbeispiels angesprochen.

Das Ausführungsbeispiel wird anhand der Zeichnung erläutert. In der Zeichnung zeigt:
- Fig. 1: eine Darstellung einer Ausführungsform eines einzelnen ersten Teilsystems,
- Fig. 2: eine Darstellung einer Ausführungsform des zweiten Teilsystems, und
- Fig. 3: eine schematische Darstellung des aus dem ersten und zweiten Teilsystem bestehenden Gesamtsystems in einer für die Blutseparation charakteristischen Anordnung, wie sie beispielsweise durch Aufhängen der verschiedenen Komponenten an einem konventionellen Ständer realisiert werden kann.

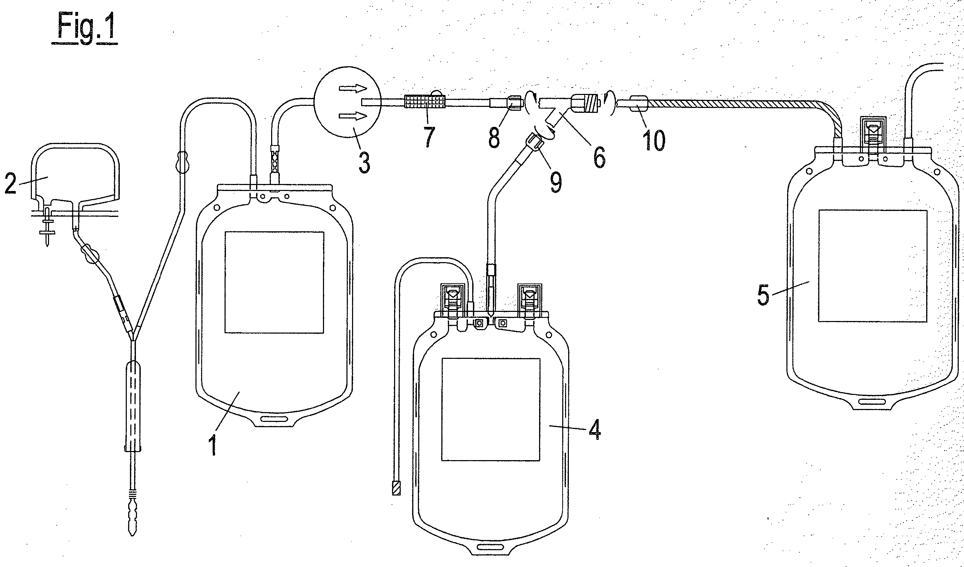

Die schematische Darstellung nach Fig. 1 zeigt die steril zu verpackenden Komponenten des ersten Teilsystems. Dieses erste Teilsystem umfasst demgemäß einen ersten Beutel 1 für das Spenderblut, einen zweiten Beutel 4 für das im Rahmen der Bluttrennung gewonnene Erythrozytenkonzentrat sowie einen dritten Beutel 5 für das bei der Bluttrennung erhaltene Plasma. Bei diesen Beuteln handelt es sich um herkömmliche flexible Beutel aus durchsichtigem Kunststoffmaterial, welche wiederum in üblieher Weise mit Zulauf- und Ablaufschläuchen und Brechventilen versehen sind, wie dies auch bei anderen Blutspendersystemen der Fall ist.

Dem zur Aufnahme von Spenderblut bestimmten ersten Beutel 1 ist in üblicher Weise ein für Testzwecke bestimmter Vorspenderbeutel 2 zugeordnet, ebenso eine übliche Spenderkanüle.

In die Ablaufleitung des ersten Beutels 1 ist im vorliegenden Fall ein herkömmlicher Leukozytenfilter 3 eingeschaltet, auf den eine Rollklemme 7 folgt. Wie ausgeführt ist das Vorhandensein eines Leukozytenfilters in der Ablaufleitung optional und nicht in jedem Fall erforderlich. Mit dem Ende des Ablaufschlauchs ist ein Male-Konnektor 8 verbunden, welcher im Rahmen des ersten Teilsystems mit einem Anschluss eines Y-Verbinders 6 gekoppelt ist. Der diesem Anschluss benachbarte Anschluss des Y-Verbinders 6 ist mit einem Male-Konnektor 9 am Ende des Anschlussschlauchs für den Erythrozytenbehälter 4 verbunden. Ein am freien Ende des Verbindungsschlauchs zum Plasmabeutel 5 vorgesehener Konnektor ist als Female-Konnektor 10 ausgebildet und mit dem verbleibenden dritten Anschluss des Y-Verbinders 6 gekoppelt.

Das in der beschriebenen Weise zusammengefasste, aus über Schläuche miteinander verbundenen Beuteln bestehende erste Teilsystem kann im verpackten Zustand durch Dampfsterilisation in herkömmlicher Weise keimfrei gemacht werden, so dass dieses erste Teilsystem in Form einer preiswerten, steril verpackten Einheit für den praktischen Einsatz für eine Blutentnahme zur Verfügung steht. In üblicher Weise wird unmittelbar nach Beendigung einer Blutspende der Zuführschlauch zum Spenderblutbeutel 1 luftdicht verschlossen, was entweder durch Verschweißen oder durch das Abklemmen des Schlauchs mit einer Metallklemme erfolgen kann.

Wie ausgeführt, können die zuvor aufgeführten Komponenten des ersten Teilsystems auch auf beispielsweise zwei erste Teilsysteme aufgeteilt sein. In diesem Fall können der erste Beutel 1 für das Spenderblut, der für Testzwecke bestimmte Vorspenderbeutel 2 sowie die Spenderkanüle zusammen mit dem Leukozytenfilter 3 und der Rollklemme 7 sowie den zugehörigen Zulauf- und Ablaufschläuchen im einen ersten Teilsystem enthalten sein. Der zweite Beutel 4 für das im Rahmen der Bluttrennung gewonnene Erythrozytenkonzentrat sowie der dritte Beutel 5 für das bei der Bluttrennung erhaltene Plasma können zusammen mit den zugehörigen Zulauf- und Ablaufschläuchen und Brechventilen Bestandteil des weiteren ersten Teilsystems sein. In diesem Fall ist eine einfache Verbindung der Beutel 4, 5 über den Male-Konnektor 8 und den Female-Konnektor 10 ausreichend und eine Verbindung über einen Y-Verbinder 6 nicht erforderlich.

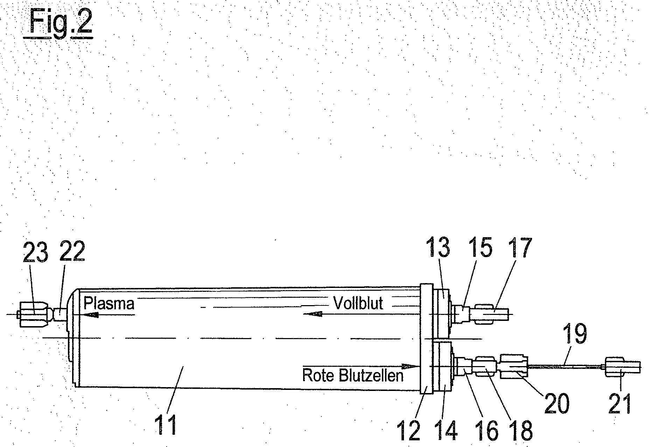

Fig. 2 zeigt in Form einer Außenansicht ein Separatormodul 11, in dessen Gehäuse ein Bündel aus einer Vielzahl von Membran-Hohlfasern angeordnet ist. Dieses Bündel kann sich in Längsrichtung durch das Gehäuse erstrecken, aber bevorzugt wird das Hohlfaserbündel wie im hier gezeigten Ausführungsbeispiel in U-Form im Gehäuse angeordnet, so dass die Zulaufseite und die Ablaufseite des Faserbündels an einem Ende des Gehäuses gelegen sind.

Das bevorzugt aus Polycarbonat bestehende, durchsichtige Gehäuse besitzt bodenseitig einen Auslaufstutzen 22 für das im Rahmen des Trennvorgangs gewonnene Plasma, und in diesen Auslaufstutzen 22 ist ein Teil-Konnektor, beispielsweise ein Male-Konnektor 23 integriert bzw. eingeklebt oder eingeschweißt. Vorzugsweise ist der Teilkonnektor integraler Bestandteil des Auslaufstutzens 22, beispielsweise indem er zusammen mit dem Auslaufstutzen 22 im Spritzguss hergestellt wurde.

Am gegenüberliegenden Ende des Separatormoduls 11 ist das Gehäuse durch eine Trägerkappe 12 verschlossen, wobei in dieser Trägerkappe bzw. einer entsprechenden Abschlussplatte die Enden der Membran-Hohlfasern fixiert sind, so dass über eine Anströmkappe 13 das Vollblut in die Innenräume der Hohlfasern einströmen kann und das die roten Blutkörperchen enthaltende Retentat nach der Trennung von dem die Hohlfaserwandungen durchdringenden Plasma über den Ablaufstutzen 16 in der Anströmkappe 14 abgeführt werden kann.

Die Anströmkappen 13, 14 sind jeweils mit einem Zulaufstutzen 15 bzw. einem Ablaufstutzen 16 ausgestattet, in die analog zu dem Auslaufstutzen 22 für das Plasma Teil-Konnektoren, vorliegend in Form von Female-Konnektoren 17, 18, unmittelbar eingesetzt und durch Verkleben oder Verschweißen dicht mit diesen Stutzen verbunden sind. Auch hier ist bevorzugt, wenn diese Teil-Konnektoren integral mit dem Zulaufstutzen 15 bzw. dem Ablaufstutzen 16 verbunden sind.

Das gemäß einer vorteilhaften Ausführungsform vorliegend als Bestandteil des zweiten Teilsystems dargestellte spezielle Anschlussstück zur Druckanpassung besteht aus einem druckfesten Schlauch 19 und an dessen Enden vorgesehenen Teil-Konnektoren 20, 21. Dieses Anschlussstück ist zur Verbindung des Separatormoduls 11 mit dem zum Erythrozytenbehälter 4 führenden Schlauch bestimmt.

Der Innendurchmesser dieses Schlauchabschnitts 19 ist geringer als der Innendurchmesser der übrigen Verbindungsschläuche. Bevorzugt liegt der Innendurchmesser dieses Schlauchabschnitts 19 bzw. allgemein des als Anschlussstück eingesetzten Leitungsabschnitts im Bereich von etwa 0,5 bis 3,5 mm, während seine Länge etwa 40 mm bis 150 mm beträgt. Durch das spezielle Anschlussstück zur Druckanpassung, hier als Schlauch- bzw. Leitungsabschnitt 19 ausgeführt, wird eine Druckerhöhung in der Ableitung zum Erythrozytenbehälter 4 bewirkt, woraus ein erhöhter Filtratfluss des Plasmas durch die Poren der Hohlfasermembranen resultiert. Als Folge davon gelangt weniger verbleibendes Plasma in den Erythrozytenbeutel. Hierdurch wird der Anteil der Flüssigkeit am Gesamtvolumen des Erythrozytenbeutels gesenkt, was eine signifikante Erhöhung des Hämatokrit-Wertes zur Folge hat.

Die unmittelbare Integration der Teil-Konnektoren in die Anschlussstutzen des Separatormoduls 11 und der dadurch erreichte Wegfall von sonst erforderlichen Verbindungs-Anschlussschläuchen auf Seiten des Separatormoduls erleichtert und vereinfacht in erheblichem Maße die Durchfühlung obligatorischen Leakage-Tests in der Serienproduktion.

Das hier dargestellte, aus dem Separatormodul 11 und dem zugehörigen Anschlussstück 19, 20, 21 bestehende zweite Teilsystem wird bevorzugt nicht dampfsterilisiert, sondern es wird eine Sterilisierung durch Gammastrahlung oder Betastrahlung vorgenommen, da auf diese Weise mit Sicherheit die geforderte Keimfreiheit des Gesamtaufbaus des aus einer Mehrzahl von Komponenten bestehenden Separatormoduls zusammen mit dem speziellen Anschlussstück gewährleistet werden kann.

Ergänzend ist darauf hinzuweisen, dass durch die gewählte Dimensionierung des Anschlussstück zur Druckanpassung, hier des Schlauchabschnitts 19 eine Erhöhung des Hämatokrit-Wertes im Erythrozytenkonzentrat um etwa 10%-Punkte gegenüber dem Ausgangs-Hämatokrit-Wert im Spenderblut sicher erreicht werden kann, also beispielsweise von 42% auf 52%.

Die offenen Teil-Konnektoren 17, 21, 23 im zweiten Teilsystem sind durch abnehmbare Abschlusskappen verschlossen. Da bevorzugt in dem Gesamtsystem als Konnektoren die bekannten Luer-Konnektoren verwendet werden, erfolgt Verschluss der Teil-Konnektoren mit passenden, vorzugsweise farbigen Caps im Luer-Lock-Verfahren.

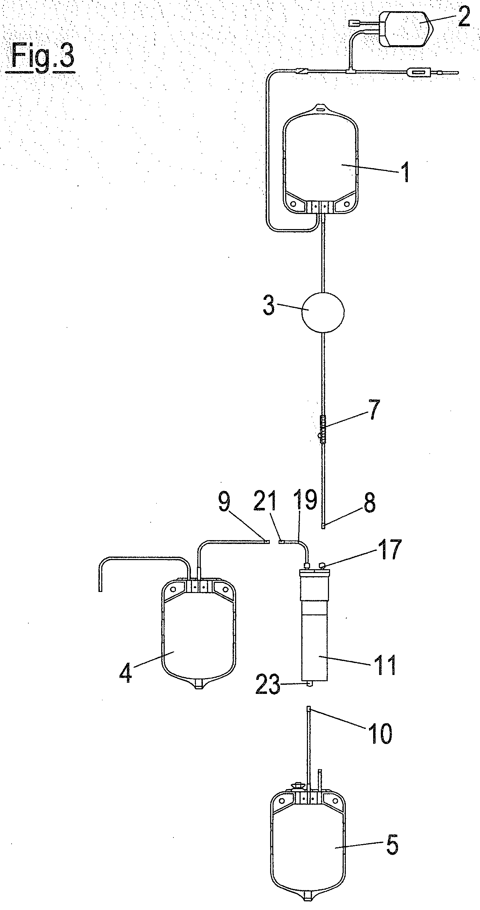

Fig. 3 zeigt die Zusammenführung der beiden Teilsysteme zum Gesamtsystem, wobei die Darstellung so gewählt ist, dass sie die grundsätzliche Positionierung der einzelnen Komponenten vertikal übereinander erkennen lässt, wie dies für das Wirksamwerden des Schwerkraftantriebs im Rahmen der Blutseparierung erforderlich ist. Die entsprechenden Komponenten werden dazu im Regelfall an einem geeigneten Ständer aufgehängt.

An oberster Stelle befindet sich der das Spenderblut enthaltende erste Beutel 1, an dessen Auslass wie üblich ein Brechventil vorgesehen ist. Gezeigt ist in dieser Darstellung auch noch der Vorspenderbeutel 2 mit Anschlussschlauch und Entnahmekanüle.

Unterhalb des Behälters 1 sind im dargestellten Fall in der als Blutzuführungsschlauch fungierenden Schlauchleitung ein herkömmlicher Leukozytenfilter 3 sowie daran anschließend eine Rollklemme 7 vorgesehen. Am Ende dieses Blut-Zuführschlauchs ist der vom Y-Verbinder 6 im ersten Teilsystem gelöste Male-Konnektor 8 vorgesehen, der unter Ausbildung einer sterilen oder weitgehend sterilen Verbindung mit dem Female-Konnektor 17 des Separatormoduls 11 gekoppelt wird.

Eine weitere sterile oder weitgehend sterile Verbindung wird zwischen dem Female-Konnektor 21 am Ende des Schlauchabschnitts 19 und dem Male-Konnektor 9 am Ende des zum zweiten Beutel 4 für das Erythrozytenkonzentrat führenden Schlauches hergestellt. Des Weiteren wird der am Boden des Separatormoduls 11 vorgesehene Male-Konnektor 23 mit dem am freien Ende des Verbindungsschlauchs zum Plasmabehälter 5 vorgesehenen Female-Konnektors hergestellt, worauf sich das Gesamtsystem in einem funktionstüchtigen, d.h. fertigen Zustand befindet.

Es ist darauf hinzuweisen, dass die Zusammenfügung von erstem und zweitem Teilsystem natürlich nur dann erfolgt, wenn der im Zusammenhang mit einer Blutspende durchgeführte Test unter Verwendung des sich im Vorspenderbeutel 2 befindenden Blutes ergibt, dass die Blutspende einwandfrei ist. Sollte dies nicht der Fall sein, muss lediglich das erste Teilsystem entsorgt werden, das aufwändigere und teurere zweite Teilsystem wird nicht beeinträchtigt und kann für die nächste durchzuführende Blutseparation verwendet werden. Mit dem erfindungsgemäßen, einfach und auch von nicht speziell ausgebildetem Personal problemfrei zu handhabenden System kann jede Einheit Spenderblut in eine Einheit Erythrozytenkonzentrat und eine Einheit Plasma separiert werden, wobei beide Komponenten zum sofortigen Gebrauch bereit sind, wo immer und wann immer sie benötigt werden.

### Bezugszeichenliste

- 1: erster Beutel bzw. Primärbeutel für Spenderblut
- 2: Vorspenderbeutel für Testzwecke
- 3: Leukozytenfilter
- 4: zweiter Beutel für das Erythrozytenkonzentrat
- 5: dritter Beutel für Plasma
- 6: Y-Verbinder
- 7: Rollklemme
- 8: Male-Konnektor
- 9: Male-Konnektor
- 10: Female-Konnektor
- 11: Separatormodul
- 12: Trägerkappe
- 13: Anströmkappe
- 14: Anströmkappe
- 15: Zulaufstutzen
- 16: Ablaufstutzen
- 17: Female-Konnektor
- 18: Female-Konnektor
- 19: Schlauchabschnitt
- 20: Male-Konnektor
- 21: Female-Konnektor
- 22: Auslaufstutzen
- 23: Male-Konnektor

## Patentansprüche

1. Mobiles System zur mittels Schwerkraft erfolgenden Aufteilung von Spenderblut in Erythrozytenkonzentrat und Plasma mit einem als Vorratsbehälter für das aufzutrennende Blut dienenden ersten Beutel (1) und zumindest einem zweiten und einem dritten Beutel (4, 5) zur Aufnahme von Erythrozytenkonzentrat bzw. Plasma, sowie mehreren Schläuchen zur Verbindung der aus Kunststoffmaterial bestehenden flexiblen Beutel mit einem eine Trennmembran in Form eines Hohlfaserbündels aufweisenden Separatormodul (11),
**dadurch gekennzeichnet,**
**dass** das System in ein mindestens erstes und ein zweites steril verpacktes, individuelles Teilsystem aufgeteilt ist,
**dass** das mindestens eine erste Teilsystem die einzelnen Beutel (1, 4, 5) und zugehörige Verbindungsschläuche und das zweite Teilsystem das Separatormodul (11) umfasst, und
**dass** zur Verbindung der Schläuche mit dem Separatormodul (11) Male-/Female-Konnektoren (8, 17; 20, 18; 10, 23), insbesondere Luer-Lock-Konnektoren, vorgesehen sind, deren einer Teil jeweils im Gehäuse des Separatormoduls (11) fixiert bzw. integriert und deren komplementärer Teil mit dem Ende des jeweiligen Schlauchs verbunden ist.

2. Mobiles System nach Anspruch 1,
**dadurch gekennzeichnet,**
**dass** die verpackten Teilsysteme mittels unterschiedlicher Verfahren sterilisiert sind, wobei bevorzugt das mindestens eine erste Teilsystem dampfsterilisiert und das zweite Teilsystem durch Gammastrahlung oder Betastrahlung sterilisiert ist.

3. Mobiles System nach Anspruch 1 oder 2,
**dadurch gekennzeichnet,**
**dass** in den steril verpackten Teilsystemen die offenen Teil-Konnektoren durch abnehmbare Passkappen verschlossen sind.

4. Mobiles System nach Anspruch 1 oder 2,
**dadurch gekennzeichnet,**
**dass** es in ein erstes und ein zweites steril verpacktes, individuelles Teilsystem aufgeteilt ist und dass das erste Teilsystem die Gesamtheit der einzelnen Beutel (1, 4, 5) und zugehörigen Verbindungsschläuche umfasst.

5. Mobiles System nach Anspruch 4,
**dadurch gekennzeichnet,**
**dass** im steril verpackten ersten Teilsystem die KonnektorAnschlüsse der Ablauf- bzw. Zulaufschläuche der Beutel (1, 4, 5) mit einem Y-Konnektor (6) über Male-/Female-Verbindungen zusammengeführt sind.

6. Mobiles System nach Anspruch 1,
**dadurch gekennzeichnet,**
**dass** das mindestens eine steril verpackte erste Teilsystem in der Ablaufleitung des Vollblutbeutels (1) einen Leukozytenfilter (3) mit einer nachgeordneten, einstellbaren Klemmvorrichtung (7) sowie einem Vorspendebeutel (2) und eine Spenderkanüle umfasst.

7. Mobiles System nach Anspruch 1,
**dadurch gekennzeichnet,**
**dass** das steril verpackte zweite Teilsystem ein beidendig mit einem Konnektorteil (20, 21) versehenes Anschlussstück zur Druckanpassung (19) umfasst, das mit einem dem Erythrozytenauslass zugeordneten komplementären Konnektorteil (18) des Separatormoduls (11) gekoppelt ist.

8. Mobiles System nach Anspruch 1,
**dadurch gekennzeichnet,**
**dass** das mindestens eine steril verpackte erste Teilsystem ein beidendig mit einem Konnektorteil (20, 21) versehenes Anschlussstück zur Druckanpassung (19) umfasst, das mit einem dem Erythrozytenauslass zugeordneten komplementären Konnektorteil (18) des Separatormoduls (11) koppelbar ist.

9. Mobiles System nach Anspruch 7 oder 8,
**dadurch gekennzeichnet,**
**dass** das Anschlussstücks zur Druckanpassung (19) ein Leitungsabschnitt mit einem Innendurchmesser im Bereich von etwa 0,5 bis 3,5 mm und einer Länge im Bereich von etwa 40 mm bis 150 mm ist.

10. Mobiles System nach einem der vorhergehenden Ansprüche,
**dadurch gekennzeichnet,**
**dass** das rohrförmige, sich in Richtung des Bodens vorzugsweise leicht verjüngende, insbesondere aus Polycarbonat bestehende Gehäuse des Separatormoduls (11) bodenseitig einen Auslassstutzen (22) für das Plasma und deckelseitig einen Zulaufstutzen (15) für das zu trennende Spenderblut sowie einen Auslassstutzen (16) für das Erythrozytenkonzentrat aufweist.

11. Mobiles System nach Anspruch 10,
**dadurch gekennzeichnet,**
**dass** in den jeweiligen Stutzen (15, 16, 22) die für die sterilen Schlauchanschlüsse erforderlichen Teil-Konnektoren durch Verkleben oder Vergießen bzw. Angießen fixiert sind und bevorzugt integraler Bestandteil des jeweiligen Stutzens (15, 16, 22) sind.

12. Mobiles System nach Anspruch 10 oder 11,
**dadurch gekennzeichnet,**
**dass** der bodenseitige Auslassstutzen (22) des Separatormoduls einen von den deckelseitigen Stutzen (15, 16) verschiedenen Teil-Konnektor aufweist.

13. Mobiles System nach einem der vorhergehenden Ansprüche,
**dadurch gekennzeichnet,**
**dass** als Konnektoren Luer-Konnektoren verwendet und Teil-Konnektoren mit passenden Caps im Luer-Lock-Verfahren verschlossen sind.

## Claims

1. Mobile system for separating donor blood by means of gravitational force into erythrocyte concentrate and plasma, having a first bag (1) serving as a reservoir for the blood to be separated and at least a second and third bag (4, 5) for respectively receiving erythrocyte concentrate and plasma, and also multiple tubes for connecting the flexible bags consisting of plastic material to a separator module (11) having a separating membrane in the form of a bundle of hollow fibers,
**characterized in that**
the system is divided into at least one first and one second sterilely packaged, individual subsystem,
the at least one first subsystem comprises the individual bags (1, 4, 5) and associated connection tubes, and the second subsystem comprises the separator module (11), and
male/female connectors (8, 17; 20, 18; 10, 23), in particular Luer-lock connectors, are provided for connecting the tubes to the separator module (11), one part of said connectors being fixed or integrated in the housing of the separator module (11) and the complementary part of said connectors being connected to the end of the respective tube.

2. Mobile system according to Claim 1,
**characterized in that**
the packaged subsystems are sterilized by means of different methods, wherein, preferably, the at least one first subsystem is steam sterilized and the second subsystem is sterilized using gamma radiation or beta radiation.

3. Mobile system according to Claim 1 or 2,
**characterized in that**
the open gender connectors in the sterilely packaged subsystems are closed off by removable caps.

4. Mobile system according to Claim 1 or 2,
**characterized in that**
it is divided into a first and a second sterilely packaged, individual subsystem, and the first subsystem comprises the entirety of the individual bags (1, 4, 5) and associated connection tubes.

5. Mobile system according to Claim 4,
**characterized in that**
in the sterilely packaged first subsystem, the connectors of the inlet and outlet tubes of the bags (1, 4, 5) are joined using a Y connector (6) via male/female connections.

6. Mobile system according to Claim 1,
**characterized in that**
the at least one sterilely packaged first subsystem comprises in the outlet line of the whole blood bag (1) a leukocyte filter (3) with a downstream, adjustable clamping device (7), as well as a pre- donation bag (2) and a donor cannula.

7. Mobile system according to Claim 1,
**characterized in that**
the sterilely packaged second subsystem comprises a pressure adapter (19) provided on both ends with a connector (20, 21), which pressure adapter is coupled to a complementary connector (18) of the separator module (11) associated with the erythrocyte outlet.

8. Mobile system according to Claim 1,
**characterized in that**
the at least one sterilely packaged first subsystem comprises a pressure adapter (19) provided on both ends with a connector (20, 21), which pressure adapter can be coupled to a complementary connector (18) of the separator module (11) associated with the erythrocyte outlet.

9. Mobile system according to Claim 7 or 8,
**characterized in that**
the pressure adapter (19) is a line section with an inside diameter in the range from approximately 0.5 to 3.5 mm and a length in the range from approximately 40 mm to 150 mm.

10. Mobile system according to one of the preceding claims,
**characterized in that**
the tubular-shaped housing of the separator module (11), preferably tapering slightly toward the bottom and in particular made of polycarbonate, has on the bottom a discharge fitting (22) for the plasma and on the cover end an inlet fitting (15) for the donor blood to be separated and an outlet fitting (16) for the erythrocyte concentrate.

11. Mobile system according to Claim 10,
**characterized in that**
the gender connectors required for the sterile tube connections are fixed by gluing them to or casting them in or on the respective fittings (15, 16, 22), and are preferably integral components of the respective fittings (15, 16, 22).

12. Mobile system according to Claim 10 or 11,
**characterized in that**
the bottom discharge fitting (22) of the separator module has a different gender connector than the cover-end fittings (15, 16).

13. Mobile system according to one of the preceding claims
**characterized in that**
Luer connectors are used as connectors and gender connectors are closed off with suitable caps in the Luer-lock procedure.

## Revendications

1. Système mobile pour la séparation, réalisée par gravité, de sang de donneur en concentré érythrocytaire et en plasma, comprenant une première poche (1) servant de réserve de sang à séparer et au moins une deuxième et une troisième poche (4, 5) pour recevoir du concentré érythrocytaire, respectivement du plasma, ainsi que plusieurs tuyaux pour relier les poches souples, constituées de matériau synthétique, à un module séparateur (11) comportant une membrane séparatrice en forme de faisceau de fibres creuses, **caractérisé en ce que** le système est divisé en un au moins premier et un second sous-système individuel conditionné stérilement, **en ce que** le au moins un premier sous-système comprend les différentes poches (1, 4, 5) et les tuyaux de liaison associés, et le second sous-système comprend le module séparateur (11), et **en ce que** pour relier les tuyaux au module séparateur (11) sont prévus des connecteurs mâle/femelle (8, 17 ; 20, 18 ; 10, 23), en particulier des connecteur Luer Lock, dont l'une des parties est fixée, respectivement intégrée au boîtier du module séparateur (11) et dont l'autre partie complémentaire est reliée à l'extrémité du tuyau correspondant.

2. Système mobile selon la revendication 1, **caractérisé en ce que** les sous-systèmes conditionnés sont stérilisés au moyen de procédés divers, de préférence le au moins un premier sous-système étant stérilisé à la vapeur et le second sous-système étant stérilisé par rayonnement gamma ou rayonnement béta.

3. Système mobile selon la revendication 1 ou 2, **caractérisé en ce que**, dans les sous-systèmes conditionnés stérilement, les connecteurs partiels ouverts sont fermés par des coiffes adaptables amovibles.

4. Système mobile selon la revendication 1 ou 2, **caractérisé en ce qu'**il est divisé en un premier et un second sous-système individuel conditionné stérilement, et **en ce que** le premier sous-système comprend la totalité des différentes poches (1, 4, 5) et les tuyaux de liaison associés.

5. Système mobile selon la revendication 4, **caractérisé en ce que**, dans le premier sous-système conditionné stérilement, les raccords de connecteur des tuyaux de sortie, respectivement d'entrée (1, 4, 5) sont reliés à un connecteur en Y (6) par l'intermédiaire de liaisons mâle-femelle.

6. Système mobile selon la revendication 1, **caractérisé en ce que** le au moins un premier sous-système conditionné stérilement comprend, dans la conduite de sortie de la poche de sang total (1), un filtre à leucocytes (3), muni d'un dispositif de serrage réglable disposé en aval (7) ainsi que d'une poche de pré-don (2), et une canule de prélèvement.

7. Système mobile selon la revendication 1, **caractérisé en ce que** le second sous-système conditionné stérilement comprend un raccord d'adaptation de pression (19) qui est muni à ses deux extrémités d'une partie de connecteur (20, 21) et qui est couplé à une partie de connecteur complémentaire (18) du module séparateur (11) associée à la sortie d'érythrocytes.

8. Système mobile selon la revendication 1, **caractérisé en ce que** le au moins un premier sous-système conditionné stérilement comprend un raccord d'adaptation de pression (19) qui est muni à ses deux extrémités d'une partie de connecteur (20, 21) et qui peut être couplé à une partie de connecteur complémentaire (18) du module séparateur (11) associée à la sortie d'érythrocytes.

9. Système mobile selon la revendication 7 ou 8, **caractérisé en ce que** le raccord d'adaptation de pression (19) est une portion de conduite possédant un diamètre intérieur situé dans une plage d'environ 0,5 à 3,5 mm et une longueur située dans une plage d'environ 40 mm à 150 mm.

10. Système mobile selon une des revendications précédentes, **caractérisé en ce que** le boîtier tubulaire du module séparateur (11), lequel se rétrécit de préférence légèrement en direction du fond et est constitué en particulier de polycarbonate, comporte, côté fond, un embout de sortie (22) pour le plasma et, côté couvercle, un embout d'entrée (15) pour le sang de donneur à séparer ainsi qu'un embout de sortie (16) pour le concentré érythrocytaire.

11. Système mobile selon la revendication 10, **caractérisé en ce que** les connecteurs partiels nécessaires aux raccords de tuyaux stériles sont fixés dans les embouts correspondants (15, 16, 22) par collage ou fusion, respectivement assemblage par fusion, et font de préférence partie intégrante de l'embout correspondant (15, 16, 22).

12. Système mobile selon la revendication 10 ou 11, **caractérisé en ce que** l'embout de sortie côté fond (22) du module séparateur comporte un connecteur partiel différent de l'embout côté couvercle (15, 16).

13. Système mobile selon une des revendications précédentes, **caractérisé en ce que** les connecteurs employés sont des connecteurs Luer, et les connecteurs partiels sont fermés par des coiffes appropriées selon le procédé Luer Lock.
